# FASCICULE DE BREVET EUROPEEN

(11) **EP 0 591 332 B1**
(45) Date de publication et mention de la délivrance du brevet: **20.12.2000**
(21) Numéro de dépôt: 92913338.7
(22) Date de dépôt: 24.06.1992
(51) Int. Cl.: C12N 15/57, C12Q 1/68

(54) **MOYENS ET PROCEDES POUR L'ETUDE DU POLYMORPHISME GENETIQUE DE L'ENZYME DE CONVERSION DE L'ANGIOTENSINE I**
VERFAHREN UND MITTEL FÜR DIE ANALYSE VON GENETISCHEM POLYMORPHISMUS DES ANGIOTENSIN-UMWANDLUNGS-ENZYMS I
METHODS AND MEANS FOR STUDYING GENETIC POLYMORPHISM IN THE ANGIOTENSIN CONVERTING ENZYME

(30) Priorité: 27.06.1991 FR 9108020
(43) Date de publication de la demande: 13.04.1994
(73) Titulaire: INSTITUT NATIONAL DE LA SANTE ET DE LA RECHERCHE MEDICALE (INSERM), 75654 Paris Cédex 13 (FR)
(72) Inventeur: SOUBRIER, Florent, F-75016 Paris (FR); HUBERT, Christine, F-92310 Sèvres (FR); CORVOL, Pierre, F-75007 Paris (FR)
(74) Mandataire: Desaix, Anne
(86) Numéro de dépôt international: FR9200574
(87) Numéro de publication internationale: WO9300360

(56) Documents cités:
- WO-A-90/03435
- WO-A-91/00354
- NUCLEIC ACIDS RESEARCH. vol. 18, no. 23, 1990, ARLINGTON, VIRGINIA US pages 6793 - 6798; M. A. BATZER: 'Structure and variability of recently inserted Alu family members'
- JOURNAL OF CLINICAL INVESTIGATIONS. vol. 86, Octobre 1990, NEW YORK US pages 1343 - 1346; B. RIGAT ET AL.: 'An insertion/deletion polymorphism in the angiotensin I-converting enzyme gene accounting for half the variance of serum enzyme levels' cité dans la demande
- ANALYTICAL BIOCHEMISTRY vol. 194, Avril 1991, NEW YORK US pages 45 - 53; J. J. LANZILLO: 'Chemiluminescent nucleic acid detection with digoxigenin-labeled probes: a model system with probes for angiotensin converting enzyme which detect less than one attomole of target DNA'

## Description

La présente invention porte sur le rôle de l'intron 16 du gène de l'enzyme de conversion de l'angiotensine (ECA) et plus particulièrement de la présence ou non d'une insertion dans cet intron d'une séquence particulière dont la présence ou l'absence pourrait être une indication d'une prédisposition à l'infarctus du myocarde.

Le clonage et le séquençage de l'acide nucléique codant pour l'ECA humaine ainsi que la détermination de la chaîne peptidique correspondant à l'ECA ont été publiés dans WO 90/03435.

Le clonage de cDNAs correspondant au gène codant pour l'enzyme de conversion (ECA) humaine a permis de l'utiliser comme sonde d'ADN pour rechercher un polymorphisme de l'ADN au niveau de ce gène chez l'homme.

L'intérêt de cette recherche de polymorphisme était multiple. D'une part, il était connu que le taux de l'ECA était stable dans le plasma et l'effet d'un allèle sur un gène majeur avait été proposé à la suite d'une étude familiale. Il était donc logique de savoir si le gène de l'ECA lui-même était responsable de l'effet du gène majeur et de connaître l'importance des différences inter-individuelles du taux d'ECA, en fonction du génotype. D'autre part, il était intéressant de disposer d'un marqueur génétique pouvant servir pour tester l'implication du gène dans certaines pathologies, telle que l'hypertension artérielle, par des études d'association ou de liaison.

Sur le plan clinique, le taux de l'ECA plasmatique a été mesuré chez 80 individus normaux et leurs génotypes ont été déterminés. Des différences significatives ont été constatées entre le taux d'ECA des individus en fonction de leur génotype de l'ECA, (Rigat B. et al (1990), Journal of Clinical Investigation, 1343-1346).

La recherche de polymorphisme a été effectuée en utilisant la méthode de southern. L'ADN de différents individus a été digéré par plusieurs enzymes de restriction. Après hybridation avec la sonde cDNA de l'ECA, il était apparent que certains fragments n'avaient pas la même taille chez tous les individus. Le fait que la différence de taille entre les fragments polymorphes était apparemment très voisine avec différentes coupures enzymatiques à conduit à la conclusion que ce polymorphisme était du à la présence ou à l'absence d'une séquence d'ADN (ci-après dénommée insertion) au sein de gène de l'ECA.

L'invention découle de la localisation de l'insertion dans l'intron 16 du gène, c'est à dire entre l'exon 16 et l'exon 17, après que la structure nucléotidique de cet intron eut elle-même été déterminée. Le séquençage de l'intron, et plus particulièrement des régions bordant l'insertion et l'insertion elle-même, ont permis aux inventeurs de déterminer la nature de l'insertion. Il d'agit d'une séquence répétitive de type Alu.

L'invention a donc pour objet des produits, notamment fragments de nucléotides découlant de cet observations, plus particulièrement des amorces utilisables dans des techniques d'amplification de gène, par exemple d'amplification par polymérisation de chaînes de type PCR (abréviation anglaise de l'expression "Polymerase Chain Reaction").

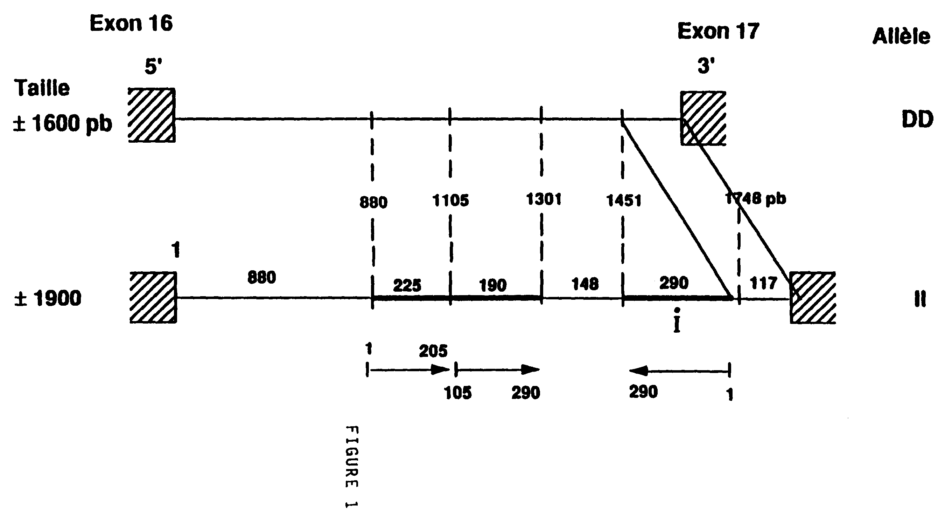

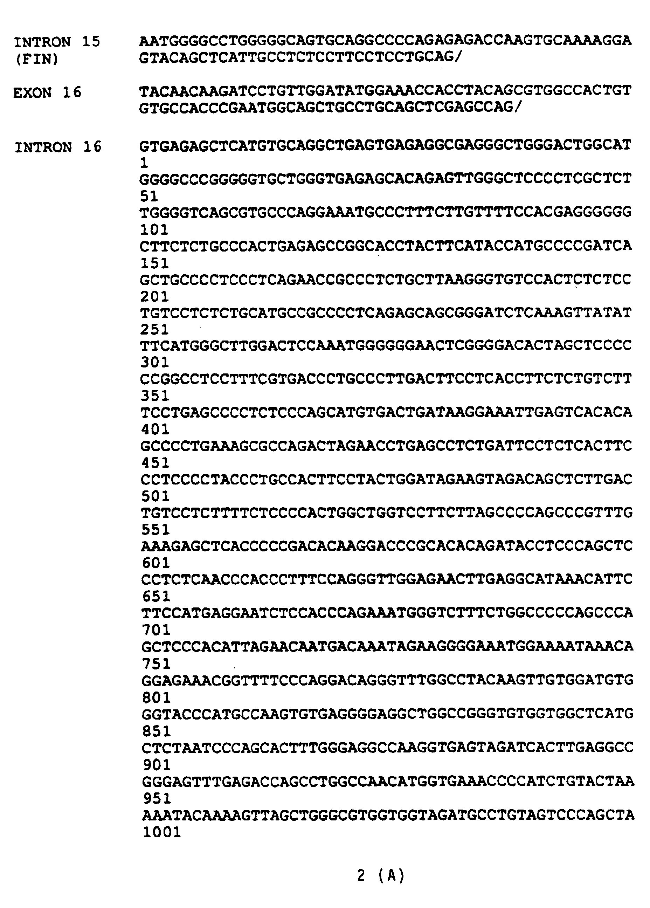

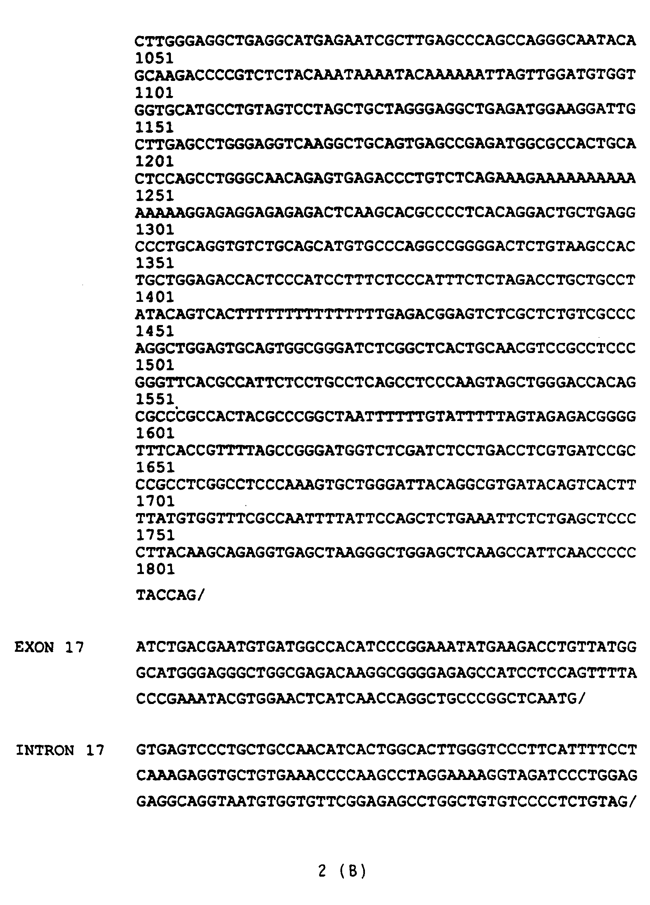

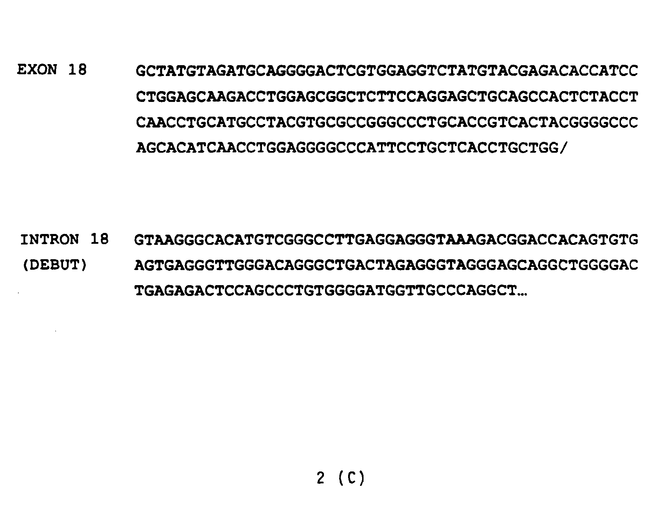

Dans ce qui suit il sera fait plus particulièrement référence à la Figure 1, relative à l'organisation générale de l'intron 16 dans des allèles provenant des sujets qui possèdent l'insertion (II) et qui en sont dépourvus (DD) respectivement. Les Figures 2(a), 2(b) et 2(c) fournissent les séquences de l'intron 16 et des exons (exons 16 et 17) qui entourent et bordent l'intron 16 dans le gène de l'ECA. Seuls les nucléotides (nt) de l'intron 16 sont numérotés.

La séquence qui est présente ou absente en fonction des individus comprend les nucléotides 1451 à 1738.

L'invention concerne donc plus particulièrement tout fragment d'ADN Comprenant de 8 à 1856 nucléotides et caractérisé par une séquence contenue dans l'ADN de l'intron 16 (nt.1-1856 de la Figure 1) le fragment compris entre les positions 1451 et 1738 étant exclu en tant que tel.

Mais elle concerne plus particulièrement l'un quelconque des fragments suivants :
- fragment d'ADN, caractérisé en ce qu'il contient l'une au moins des régions bordant la susdite insertion.
- fragment d'ADN, caractérisé en ce que sa séquence est extérieure à la séquence (insertion) nt.1451-1738.

Avantageusement ces divers fragments d'ADN comportent de 15 à 40 nucléotides.

L'invention est plus particulièrement relative à des paires de fragments distincts choisis parmi les précédents, applicables à la constitution d'amorces utilisables dans des procédés d'amplification de séquence, notamment du type PCR, ces fragments distincts comportant des séquences non chevauchantes de nucléotides.

Ces paires d'amorces sont, par exemple :
- caractérisées en ce que leurs propres séquences sont telles que la séquence intermédiaire placée dans l'ADN de l'intron entre les deux séquences qui, au sein de ce même intron, correspondent à celles de ces deux amorces, chevauche au moins l'une des extrémités (nt.1451 ou 1738) de l'insertion (nt.1451-1738) et la région bordante qui la jouxte.
- caractérisée en ce que la séquence de l'une des deux amorces chevauche l'une des extrémités (nt.1451 ou 1738) de l'insertion ou est extérieure à celle-ci et que la séquence de l'autre amorce chevauche l'autre extrémité (nt.1738 ou 1451) de l'insertion ou est également extérieure à celle-ci.
- caractérisée en ce que la séquence de l'une des deux amorces chevauche l'une des extrémités (nt.1451 ou 1738) de l'insertion ou est extérieure à celle-ci et que la séquence de l'autre amorce est incluse dans l'insertion.

L'invention concerne donc plus particulièrement le procédé de mise en évidence d'un polymorphisme au sein du gène de l'enzyme de conversion de l'angiotensine 1 (gène ECA), caractérisé par la recherche, notamment par des techniques d'hybridation ou d'amplification de séquences, et la détection de la présence ou non de l'insertion nt.1451-1738 au sein de l'intron 16 du gène ECA. On met, par exemple, en oeuvre une technique du type PCR, mettant en jeu l'une des paires d'amorces définies ci-dessous et l'on procède à la détection ou non de chaînes amplifiées susceptibles de comprendre à la fois au moins l'une des parties terminales de l'insertion et au moins d'une des deux régions bordantes.

Une détection positive manifeste alors de la présence de l'insertion, tandis qu'un résultat négatif signe l'absence d'une telle insertion.

Il va de soi que l'homme du métier peut alors recourir à toute technique classique pour avoir accès à l'ADN génomique des cellules provenant du sujet (notamment du sang) dont le polymorphisme doit être étudié.

Le procédé selon l'invention peut,par exemple, être mis en oeuvre comme suit. Il comprend :
a) la mise en contact, en présence de nucléosides triphosphates et d'un agent inducteur de polymérisation (polymérase), de l'échantillon biologique étudié et contenant le gène ECA, notamment l'intron 16 préalablement rendu accessible à une première amorce dans des conditions autorisant l'hybridation entre cette amorce et l'ADN du gène, et la polymérisation à partir de ces amorces hybridées à l'ADN du gène, pour - le cas échéant - produire un duplex formé entre le produit d'allongement de l'amorce, et la séquence servant de matrice,
b) la dénaturation du duplex obtenu à l'étape a) de façon à "séparer" le produit d'allongement de l'amorce de l'ADN à détecter,
c) la mise en contact du produit d'allongement obtenu avec la deuxième amorce ayant une séquence de nucléotides (1) non complémentaire de celle de la première amorce et (2) complémentaire d'une séquence du produit d'allongement précédemment formé,
d) le cas échéant la répétition des étapes a) et b) et c) en présence des première et deuxième amorces utilisées en excès et des réactifs nécessaires à la production de nouveaux produits d'allongement utilisés à leur tour comme matrices pour des synthèses supplémentaires, jusqu'à obtenir une quantité suffisante pour être détectée de produits d'allongement des amorces utilisées.
e) la détection (le cas échéant) de la présence des produits d'allongement caractéristiques de la présence d'une partie au moins de l'insertion (nt.1451-1738).

Pour plus de détails concernant la technique relative au procédé de détection décrit ci-dessus, ou pour l'élaboration de variantes de ce procédé, l'homme du métier pourra se reporter utilement aux principes décrits dans les brevets US.4.683.202 et US.4.683.195.

Grâce à l'utilisation de deux amorces situées de part et d'autre d'au moins une partie de la région polymorphe, il est possible de visualiser facilement la différence de taille due à la présence ou non de l'insertion, par exemple par migration sur gel d'agarose et coloration au bromure d'ethidium des fragments amplifiés, d'où la possibilité de déterminer les génotypes des individus à l'étude par la réaction d'amplification de l'ADN.

L'invention fournit par conséquent un procédé pour la détermination du polymorphisme sur les valeurs normales et anormales de l'ECA, notamment pour l'étude de son impact sur des affections (par exemple chez des individus diabétiques ou ayant une sarcoïdose) qui, selon des observations déjà faites, étaient accompagnées d'un accroissement des taux plasmatiques d'ECA.

De même, les résultats susceptibles d'être acquis par la mise en oeuvre du procédé selon l'invention devraient permettre une meilleure compréhension du degré d'implication du polymorphisme de l'ECA dans le métabolisme des peptides vasoactifs et dans les mécanismes de l'hypertension.

Le procédé selon l'invention s'applique avec un avantage particulier au diagnostic in vitro de l'athérosclérose, de maladies granulomateuses et de complications du diabète sucré.

## Revendications

1. Fragment d'ADN comprenant au moins 8 nucléotides, constitué totalement ou partiellement d'une séquence contenue dans l'ADN de l'intron 16 (n.1-1856 de la figure 2), à l'exception de tout fragment compris entre les positions 1451 à 1738 et utilisable comme sonde d'hybridation ou comme amorce d'amplification pour détecter un polymorphisme du gène de l'ECA.

2. Fragment d'ADN selon la revendication 1, caractérisé en ce qu'il contient l'une au moins des régions bordant la séquence (insertion) nt. 1451-1738.

3. Fragment d'ADN selon la revendication 1, caractérisé en ce que sa séquence est extérieure à la séquence (insertion) nt. 1451-1738.

4. Fragment d'ADN selon l'une quelconque des revendications 1 à 3, caractérisé en ce qu'il comporte de 15 à 40 nucléotides.

5. Paire de fragments distincts respectivement conformes à l'une ou plusieurs des revendications 1 à 4, applicables à la constitution d'amorces utilisables dans des procédés d'amplification de séquence, notamment du type PCR, ces fragments distincts comportant des séquences non chevauchantes de nucléotides.

6. Paire d'amorces selon la revendication 5, caractérisée en ce que leurs propres séquences sont telles que la séquence intermédiaire placée dans l'ADN de l'intron entre les deux séquences qui, au sein de ce même intron, correspondent à celles de ces deux amorces, chevauche au moins l'une des extrémités (nt. 1451 ou 1738) de l'insertion (nt. 1451-1738) et la région bordante qui la jouxte.

7. Paire d'amorces selon la revendication 4, caractérisée en ce que la séquence de l'une des deux amorces chevauche l'une des extrémités (nt. 1451 ou 1738) de l'insertion ou est extérieure à celle-ci et que la séquence de l'autre amorce chevauche l'autre extrémité (nt. 1738 ou 1451) de l'insertion ou est également extérieure à celle-ci.

8. Paire d'amorces selon la revendication 4, caractérisée en ce que la séquence de l'une des deux amorces chevauche l'une des extrémités (nt. 1451 ou 1738) de l'insertion ou est extérieure à celle-ci et que la séquence de l'autre amorce est incluse dans l'insertion.

9. Procédé de mise en évidence d'un polymorphisme au sein du gène de l'enzyme de conversion de l'angiotensine 1 (gène ECA), caractérisé par la recherche, notamment par des techniques d'hybridation ou d'amplification de séquences, et la détection de la présence ou non de l'insertion nt. 1451-1738 au sein de l'intron 16 du gène ECA.

10. Procédé selon la revendication 9, caractérisé par la mise en oeuvre d'une technique d'amplification de séquences, notamment du type PCR, mettant en jeu une paire d'amorces selon l'une quelconque des revendications 6 à 8, et par la détection ou non de chaînes amplifiées susceptibles de comprendre à la fois au moins l'une des parties terminales de l'insertion et au moins d'une des deux régions bordantes.

## Claims

1. DNA fragment comprising at least 8 nucleotides, constituted wholly or in part by a sequence contained in the DNA of the intron 16 (nt. 1-1856 of Figure 2) with the exception of any fragment included between the positions 1451 and 1738 and utilisable as a hybridization probe or as amplifying primer to detect a polymorphism of the ACE gene.

2. DNA fragment according to Claim 1, characterized in that it contains at least one of the regions flanking the sequence (insertion) nt. 1451-1738.

3. DNA fragment according to Claim 1, characterized in that its sequence lies outside the sequence (insertion) nt. 1451-1738.

4. DNA fragment according to any one of the Claims 1 to 3, characterized in that it contains from 15 to 40 nucleotides.

5. Pair of distinct fragments conforming respectively to one or more of the Claims 1 to 4, which suitable for constituting primers which can be used in sequence amplification procedures, in particular of the PCR type, these distinct fragments containing non-overlapping nucleotide sequences.

6. Pair of primers according to Claim 5, characterized in that their intrinsic sequences are such that the intermediate sequence placed in the DNA of the intron between the two sequences which, within the same intron, correspond to those of these two primers, overlaps at least of the ends (nt.1451 or 1738) of the insertion (nt.1451-1738) and the flanking region which is adjacent to it.

7. Pair of primers according to Claim 4, characterized in that the sequence of one of the two primers overlaps one of the ends (nt.1451 or 1738) of the insertion or lies outside the latter and in that the sequence of the other primer overlaps the other end (nt.1738 or 1451) of the insertion or also lies outside it.

8. Pair of primers according to Claim 4, characterized in that the sequence of one of the two primers overlaps one of the ends (nt.1451 or 1738) of the insertion or lies outside the latter and in that the sequence of the other primer is included in the insertion.

9. Process for the detection of a polymorphism within the gene for the angiotensin I converting enzyme (ACE gene), characterized by the search for, in particular by hybridization or sequence amplification techniques, and the detection of the presence or absence of the insertion nt.1451-1738 within the intron 16 of the ACE gene.

10. Process according to Claim 9, characterized by the use of a sequence amplification technique, in particular of the PCR type, employing a pair of primers according to any one of the claims 6 to 8, and by detection of or failure to detect amplified chains likely to contain both at least one of the terminal parts of the insertion and at least one of the two flanking regions.

## Patentansprüche

1. DNA-Fragment, umfassend mindestens 8 Nukleotide, bestehend ganz oder teilweise aus einer Sequenz, die in der DNA von Intron 16 (Nr. 1-1856 der Figur 2) enthalten ist, ausgenommen jedes Fragment zwischen den Positionen 1451 bis 1738, und verwendbar als Hybridisierungssonde oder als Amplifikationsstarter zum Nachweis eines Genpolymorphismus von ECA.

2. DNA-Fragment nach Anspruch 1, dadurch **gekennzeichnet**, dass es mindestens eine der Regionen, die an die Sequenz (Insertion) Nukleotid 1451-1738 angrenzen, enthält.

3. DNA-Fragment nach Anspruch 1, dadurch **gekennzeichnet**, dass dessen Sequenz außerhalb der Sequenz (Insertion) Nukleotid 1451-1738 liegt.

4. DNA-Fragment nach einem der Ansprüche 1 bis 3, dadurch **gekennzeichnet**, dass es 15 bis 40 Nukleotide umfasst.

5. Paar von jeweils unterschiedlichen Fragmenten nach einem oder mehreren der Ansprüche 1 bis 4, anwendbar zur Bildung von Startern, die in Sequenzamplifikationsverfahren ,insbesondere vom PCR-Typ, verwendbar sind, wobei diese unterschiedlichen Fragmente nicht überlappende Nukleotidssequenzen umfassen.

6. Starterpaar nach Anspruch 5, dadurch **gekennzeichnet**, dass ihre eigenen Sequenzen so sind, dass die Intermediär-Sequenz in der DNA des Introns zwischen den beiden Sequenzen vorhanden ist, die in diesem gleichen Intron denjenigen dieser zwei Starter entsprechen, wobei mindestens eines der Enden (Nukleotid 1451 oder 1738) der Insertion (Nukleotid 1451-1738) und der daran angrenzenden benachbarten Region überlappen.

7. Starterpaar nach Anspruch 4, dadurch **gekennzeichnet**, dass die Sequenz einer der zwei Starter mit einem der Enden (Nukleotid 1451 oder 1738) der Insertion überlappt oder außerhalb davon liegt und dass die Sequenz des anderen Starters mit dem anderen Ende (Nukleotid 1451 oder 1738) der Insertion überlappt oder auch außerhalb davon liegt.

8. Starterpaar nach Anspruch 4, dadurch **gekennzeichnet**, dass die Sequenz einer der zwei Starter mit einem der Enden (Nukleotid 1451 oder 1738) der Insertion überlappt oder außerhalb davon liegt und dass die Sequenz des anderen Starters in der Insertion eingeschlossen ist.

9. Verfahren zum Nachweis eines Polymorphismus im Gen des Angiotensin 1 umwandelnden Enzyms (ECA-Gen), dadurch **gekennzeichnet**, dass man insbesondere mit Hybridisierungs- oder Sequenzamplifikationstechniken absucht und die Anwesenheit oder das Fehlen der Insertion Nukleotid 1451-1738 im Inneren des Introns 16 des ECA-Gens nachweist.

10. Verfahren nach Anspruch 9, dadurch **gekennzeichnet**, dass man eine Sequenzamplifikationstechnik, insbesondere vom PCR-Typ, verwendet, bei der ein Starterpaar nach einem der Ansprüche 6 bis 8 gleichzeitig mindestens eine der Endpartien der Insertion und mindestens eine der zwei angrenzenden Regionen umfassen können, nachweist oder nicht.
